# EUROPÄISCHE PATENTSCHRIFT

(11) **EP 0 305 902 B1**
(45) Veröffentlichungstag und Bekanntmachung des Hinweises auf die Patenterteilung: **19.01.1994**
(21) Anmeldenummer: 88113894.5
(22) Anmeldetag: 25.08.1988
(51) Int. Cl.: A61K 31/245, A61K 31/375

(54) **Arzneimittel**
Medicament
Médicament

(30) Priorität: 01.09.1987 DE 3729201; 12.01.1988 DE 3800637
(43) Veröffentlichungstag der Anmeldung: 08.03.1989
(73) Patentinhaber: SILOR HOLDING S.A., Panama 5 (PA)
(72) Erfinder:
(74) Vertreter: Werner, Hans-Karsten, Dr.Dipl.-Chem.

(56) Entgegenhaltungen:
- BOLL.SOC. ITAL. FARM. OSP., Band 21, 1975, Seiten 461-465, IT; C. RIVA: "Herpes simplex e terapia vaccinica"
- CHEMICAL ABSTRACTS, Band 78, Nr. 26, 2. Juli 1973, Seite 290, Zusammenfassung Nr. 164122c, Columbus, Ohio, US; G.S. ORLOV: & SU-A-363 493 (KHARKOV SCIENTIFIC-RESEARCH INSTITUTE OF PROSTHETICS, AND TRAUMATOLOGY) 25-12-1972
- "Zeitschrift Raum und Zeit ", Ausgaben 40/89 und 41/89 Abstract Form, Sixth International Conference on AIDS, San Francisco, California (USA) 1990

## Beschreibung

Die Erfindung betrifft die Verwendung eines Gemisches aus Procain und Lidocain zur Herstellung eines Arzneimittels.

In der medizinischen Literatur der letzten Jahre werden erhöhte Cortisol-Spiegel als Ursache vieler Gesundheitsstörungen und Krankheiten mit bislang unbekannter Ursache genannt. Weiter ist bekannt, daß Cortisol als Immunsuppressor wirkt (vgl. Ullmann, 4. Auflage, Band 13, S.47 ff.; Sapse A.T., Medical Hypothesis, Vol.13, 31-44, 1984; Da Prato R.A., Rothschild J., The AIDS-Virus as an opportunistic organism inducing a state of chronic relative cortisol excess, Medical Hypothesis Nov. 1986, 153-266).

Durch Cortisol wird insbesondere die körpereigene Produktion von Interferonen unterdrückt. Weiterhin werden selektiv die T-Lymphozyten beeinflußt und der Spiegel der T-Helferzellen reduziert, während der Spiegel der T-Suppressor-Zellen erhöht und die Produktion von natürlichen Killer-Zellen unterdrückt wird. All diese von Cortisol ausgelösten Reaktionen führen zu einer Unterdrückung von Teilen des Immunsystems.

Von den zahlreichen Symptomen, die bei erhöhtem Cortisol-Spiegel auftreten können, seien beispielsweise Depression, Angst, Spannungszustände, Melancholie, Unruhe, Schwindel und Nervosität genannt.

Zu den durch erhöhte Cortisol-Spiegel verursachten Krankheiten gehören beispielsweise Angina pectoris, Colitis ulcerosa, Parkinsonismus, Ulcus duodeni et ventriculi, Anorexia nervosa und Virusinfektionen. Chronisch erhöhte Cortisol-Spiegel bewirken ferner ein vorzeitiges Altern.

Aus der medizinischen Literatur geht ferner hervor, daß erhöhte Cortisol-Spiegel das Resultat von Stress-Belastungen sind, weshalb Cortisol auch das "Stress-Hormon" genannt wird. Unter Stress-Bedingungen wird rund die 10-fache Menge Cortisol produziert, ein im heutigen Leben alltäglicher Vorgang. Diese unter Stress ausgeschütteten Cortisol-Mengen entsprechen bis zu 100 mg und mehr pro Tag an Cortison. Welche negativen Wirkungen solche Cortison-Mengen aufweisen, ist ebenfalls bekannt.

Der Erfindung liegt daher die Aufgabe zugrunde, eine neue Verwendung zur Herstellung eines Arzneimittels zu schaffen, die in der Lage ist, erhöhte Cortisol-Spiegel im Körper zu senken, und die für die Behandlung von AIDS geeignet ist.

Diese Aufgabe wird erfindungsgemäß durch die Merkmale des Anspruches 1 gelöst. Zweckmäßige Ausgestaltungen der Erfindung sind Gegenstand der Unteransprüche.

Bei den der Erfindung zugrundeliegenden Versuchen hat sich gezeigt, daß durch ein Gemisch von Procain und Lidocain innerhalb eines kurzen Zeitraums ein zuvor erhöhter Cortisol-Spiegel um ca. 80 % abgesenkt wird. Überraschenderweise wirkt die Kombination beider Bestandteile dahingehend, daß der sonst übliche enzymatische Abbau, der etwa zwanzig Minuten dauert, auf mehrere Stunden verlängert wird. Die Hydrolyse im menschlichen Serum wird also bei gleichzeitiger Gabe von Lidocain und Procain verzögert. Dabei liegen zweckmäßig beide Stoffe als Hydrochloride vor.

Die gleichzeitige Gabe von Lidocain und Procain hat noch einen weiteren Vorteil: Procain ist ein nervocerebrales Stimulans, da es als Monoaminooxydase-Hemmer wirkt. Es erhöht ferner die Sauerstoffutilisation durch die Gehirnzellen, und sein Abbauprodukt, das Diäthylaminoäthanol, wirkt als Zellmembranstabilisator.

Nun vertragen nicht alle Personen Procain, das bei manchen Patienten zu Übelkeit, Magenbeschwerden, Herzjagen usw. führen kann. Die gleichzeitige Gabe von Procain und Lidocain vermeidet diese unerwünschten Nebeneffekte, ebenso eine zu starke neurocerebrale Stimulierung, die häufig zu Schlaflosigkeit führt.

Lidocain trägt wesentlich zur Senkung eines zuvor erhöhten Cortisol-Spiegels bei. Es wirkt hierbei als mildes Sedativum und beseitigt Angst und Spannungen.

Gemäß einer zweckmäßigen Ausgestaltung der Erfindung wird dem Gemisch von Procain und Lidocain Ascorbinsäure zugegeben, die bei oralen Gaben die Verträglichkeit erhöht und bei parenteraler Gabe den pH-Wert des Gewebes erniedrigt, womit die Hydrolyse von Lidocain und Procain weiter verzögert wird. In der gleichen Richtung wirkt auch die Zugabe von Pantothensäure. Bei Zumischung der beiden Vitamine kommt es sogar zur Bildung gewisse Komplexe, die ebenfalls stabilisierend wirken.

Eine weitere zweckmäßige Ausgestaltung der Erfindung sieht vor, daß das Gemisch außer Procain und Lidocain zusätzlich einen aus den Peyer'schen Plaques hergestellten Organextrakt enthält.

Zur oralen Anwendung kann das Arzneimittel beispielsweise aus 10 g Ascorbinsäure 2 g Procainhydrochlorid, 2 g Lidocainhydrochlorid und 1 g Pantothensäure-Salz (Ca- oder Na-Salz) bestehen. Anschließend wird das Gemisch in vorzugsweise dünndarmlösliche Kapseln derart abgefüllt, daß jede Kapsel 100 mg Procainhydrochlorid und 100 mg Lidocainhydrochlorid zusammen mit den anderen Bestandteilen enthält.

Zur parenteralen Anwendung besteht das Arzneimittel zweckmäßig erweise aus einem Gemisch von 3 ml 2%iger Procainhydrochloridlösung und 3 ml 2%iger Lidocainhydrochloridlösung 1 g Ascorbinsäure, 250 mg Pantothenol, und 3 ml Wasser. Hierbei kann ferner noch 1 ml einer 20%igen wässrigen Magnesiumchloridlösung enthalten sein.

Ein weiteres Ausführungsbeispiel sieht vor, daß das Arzneimittel aus 100 mg lyophilisiertem Trockenorganextrakt aus den Peyer'schen Plaques, 10 ml 2%iger Lidocainhydrochloridlösung und 10 ml 2%iger Procainhydrochloridlösung besteht. Es können ferner zusätzlich 500 mg Ascorbinsäure in dem zu injizierenden Arzneimittel enthalten sein.

Eingehende Untersuchungen ergaben ferner, daß das Arzneimittel, insbesondere die Kombination von Procain, Lidocain und einem lyophilisierten Gesamtextrakt der Peyer'schen Plaques, mit Erfolg bei AIDS-Kranken, insbesondere der Grade Walter Reed 1 bis 3, angewendet werden kann.

## Patentansprüche

1. Verwendung eines Gemisches aus Procain und Lidocain zur Herstellung eines Arzneimittels zur Absenkung eines erhöhten Cortisol-Spiegels sowie zur Behandlung von AIDS.

2. Verwendung eines Gemisches nach Anspruch 1, das aus 50% Procain und 50% Lidocain besteht.

3. Verwendung eines Gemisches nach Anspruch 1, das zusätzlich Ascorbinsäure enthält.

4. Verwendung eines Gemisches nach Anspruch 3, das weiterhin Pantothensäure enthält.

5. Verwendung eines Gemisches nach Anspruch 4 zur Herstellung eines oral zu applizierenden Arzneimittels, das aus 10 g Ascorbinsäure, 2 g Procainhydrochlorid, 2 g Lidocainhydrochlorid und 1 g Pantothensäure-Salz (Ca- oder Na-Salz) besteht.

6. Verwendung eines Gemisches nach Anspruch 5, dadurch gekennzeichnet, daß das Arzneimittel in dünndarmlöslichen Kapseln vorliegt, wobei jede Kapsel außer den übrigen Bestandteilen 100 mg Procainhydrochlorid und 100 mg Lidocainhydrochlorid enthält.

7. Verwendung eines Gemisches nach Anspruch 4 zur Herstellung eines parenteral zu applizierenden Arzneimittels, dadurch gekennzeichnet, daß das Arzneimittel aus 3 ml 2% iger Procainhydrochloridlösung, 3 ml 2%iger Lidocainhydrochloridlösung, 1 g Ascorbinsäure, 250 mg Pantothenol und 3 ml Wasser besteht.

8. Verwendung nach Anspruch 7, dadurch gekennzeichnet, daß das Arzneitmittel zusätzlich noch 1 ml einer 20%igen wässrigen Magnesiumchloridlösung enthält.

9. Verwendung eines Gemisches nach Anspruch 1, dadurch gekennzeichnet, daß das Arzneimittel zusätzlich noch einen aus den Peyer'schen Plaques hergestellten Organgesamtextrakt enthält.

10. Verwendung eines Gemisches nach Anspruch 9, dadurch gekennzeichnet, daß das Arzneimittel aus 100 mg lyophilisiertem Trockenorganextrakt aus den Peyer'schen Plaques 10 ml 2%iger Lidocainhydrochloridlösung und 10 ml 2%iger Procainhydrochloridlösung besteht.

11. Verwendung eines Gemisches nach Anspruch 10, dadurch gekennzeichnet, daß das gegebenenfalls zu injizierende Arzneimittel zusätzlich 500 mg Ascorbinsäure enthält.

12. Arzneimittel zur Absenkung eines erhöhten Cortisol-Spiegels sowie zur Behandlung von AIDS, dadurch gekennzeichnet, daß es ein Gemisch aus Procain, Lidocain und einen aus den Peyer'schen Plaques hergestellten Organgesamtextrakt enthält.

13. Arzneimittel gemäß Anspruch 12, dadurch gekennzeichnet, daß es außerdem noch Ascorbinsäure und/oder Pantothenol enthält.

## Claims

1. Use of a mixture of Procaine and Lidocaine for the preparation of a medicament for lowering an elevated cortisol level as well as for the treatment of AIDS.

2. Use of a mixture according to claim 1, said mixture consisting of 50% of Procaine and 50% of Lidocaine.

3. Use of a mixture according to claim 1, said mixture additionally containing ascorbic acid.

4. Use of a mixture according to claim 3, said mixture further containing pantothenic acid.

5. Use of a mixture according to claim 4 for the preparation of a medicament for oral application, said medicament consisting of 10 g of ascorbic acid, 2 g of Procaine hydrochloride, 2 g of Lidocaine hydrochloride, and 1 g of a pantothenic acid salt (Ca or Na salt).

6. Use of a mixture according to claim 5, characterized in that the medicament is in the form of capsules which are soluble in the small intestine, where each capsule contains 100 mg of Procaine hydrochloride and 100 mg of Lidocaine hydrochloride in addition to the other components.

7. Use of a mixture according to claim 4 for the preparation of a medicament for parenteral application, characterized in that said medicament consists of 3 ml of a 2% Procaine hydrochloride solution, 3 ml of a 2% Lidocaine hydrochloride solution, 1 g of ascorbic acid, 250 mg of pantothenol and 3 ml of water.

8. Use according to claim 7, characterized in that the medicament additionally contains 1 ml of a 20% aqueous magnesium chloride solution.

9. Use of a mixture according to claim 1, characterized in that the medicament additionally contains a whole organ extract prepared from Peyer's plaques.

10. Use of a mixture according to claim 9, characterized in that the medicament consists of 100 mg of a lyophilized dry organ extract from Peyer's plaques, 10 ml of a 2% Lidocaine hydrochloride solution and 10 ml of a 2% Procaine hydrochloride solution.

11. Use of a mixture according to claim 10, characterized in that the optionally injectable medicament additionally contains 500 mg of ascorbic acid.

12. A medicament for lowering an elevated cortisol level as well as for the treatment of AIDS, characterized in that it contains a mixture of Procaine and Lidocaine and a whole organ extract prepared from Peyer's plaques.

13. The medicament according to claim 12, characterized in that it additionally contains ascorbic acid and/or pantothenol.

## Revendications

1. Utilisation d'un mélange de procaïne et de lidocaïne pour la préparation d'un médicament pour abaisser un taux augmenté de cortisol, ainsi que pour le traitement du SIDA.

2. Utilisation d'un mélange selon la revendication 1, qui est composé de 50 % de procaïne et 50 % de lidocaïne.

3. Utilisation d'un mélange selon la revendication 1, qui contient, en outre, de l'acide ascorbique.

4. Utilisation d'un mélange selon la revendication 3, qui contient aussi de l'acide pantothénique.

5. Utilisation d'un mélange selon la revendication 4 pour la préparation d'un médicament à administrer oralement, qui est composé de 10 g d'acide ascorbique, 2 g de chlorhydrate de procaïne, 2 g de chlorhydrate de lidocaïne et 1 g de sel d'acide pantothénique (sel de calcium ou sel de sodium).

6. Utilisation d'un mélange selon la revendication 5, caractérisée en ce que le médicament est contenu dans des capsules solubles dans l'intestin grêle, chaque capsule contenant 100 mg de chlorhydrate de procaïne et 100 mg de chlorhydrate de lidocaïne, en plus des autres constituants.

7. Utilisation d'un mélange selon la revendication 4 pour la préparation d'un médicament à administrer par voie parentérale, caractérisée en ce que le médicament est composé de 3 ml d'une solution de chlorhydrate de procaïne à 2 %, 3 ml d une solution de chlorhydrate de lidocaïne à 2 %, 1 g d'acide ascorbique, 250 mg de panthothénol et 3 ml d'eau.

8. Utilisation selon la revendication 7, caractérisée en ce que le médicament contient, en outre, 1 ml d'une solution aqueuse contenant 20 % de chlorure de magnésium.

9. Utilisation d'un mélange selon la revendication 1, caractérisée en ce que le médicament contient, en outre, un extrait d organe préparé à partir des plaques de Peyer.

10. Utilisation d'un mélange selon la revendication 9, caractérisée en ce que le médicament est composé de 100 mg d'extrait d'organe sec lyophilisé obtenu à partir des plaques de Peyer, 10 ml d'une solution de chlorhydrate de lidocaïne à 2 % et 10 ml d'une solution de chlorhydrate de procaïne à 2 %.

11. Utilisation d'un mélange selon la revendication 10, caractérisée en ce que le médicament devant être éventuellement injecté contient, en outre, 500 mg d'acide ascorbique.

12. Médicament pour abaisser un taux augmenté de cortisol ainsi que pour le traitement du SIDA, caractérisé en ce qu'il contient un mélange de procaïne, de lidocaïne et d'un extrait entier d'organe préparé à partir des plaques de Peyer.

13. Médicament selon la revendication 12, caractérisé en ce qu'il contient, en outre, de l'acide ascorbique et/ou du pantothénol.
